# EUROPÄISCHE PATENTSCHRIFT

(11) **EP 1 174 180 B1**
(45) Veröffentlichungstag und Bekanntmachung des Hinweises auf die Patenterteilung: **24.01.2007**
(21) Anmeldenummer: 01116912.5
(22) Anmeldetag: 11.07.2001
(51) Int. Cl.: A61K 8/06, B01F 17/00

(54) **Feinemulsionen**
Fine emulsions
Emulsions fines

(30) Priorität: 21.07.2000 DE 10035930
(43) Veröffentlichungstag der Anmeldung: 23.01.2002
(73) Patentinhaber: Clariant Produkte (Deutschland) GmbH, 65929 Frankfurt am Main (DE)
(72) Erfinder: Miller, Dennis, Dr., 65779 Kelkheim (DE); Henning, Torsten, Dr., 65779 Kelkheim (DE); Johannpeter, Wiebke, 61449 Steinbach (DE); Wiener, Eva-Maria, 65719 Hofheim-Diedenbergen (DE)

(56) Entgegenhaltungen:
- EP-A- 0 490 053
- EP-A- 0 669 125
- EP-A- 1 029 586
- DE-A- 19 509 079
- DE-A- 19 642 090

## Beschreibung

Die Erfindung betrifft ein Verfahren zur Herstellung von Feinemulsionen, enthaltend Sorbitolester, Polyglacetolester, Sorbitanester, Fettsäureester und/oder Dimethiconcopolyole.

Feinemulsionen bzw. Mikroemulsionen sind niedrigviskose, optisch transparente Dispersionen aus zwei miteinander nicht mischbaren Flüssigkeiten, die durch mindestens ein ionisches oder nichtionisches Tensid stabilisiert sind. Bei Feinemulsionen liegen die Teilchendurchmesser im Bereich von ca. 0,1 bis 10 Mikrometer. Die Grenzflächenspannung zwischen den beiden Phasen ist extrem gering.
Die Viskosität vieler Feinemulsionen vom O/W-Typ ist vergleichbar mit der des Wassers.
Im Gegensatz zu den Feinemulsionen besitzen die sogenannten Makroemulsionen hohe Viskositäten und ihr Teilchendurchmesser liegt im Bereich von ca. 10 bis 100 Mikrometer. Makroemulsionen sind milchigweiß gefärbt und neigen bei Temperaturbelastung zur Phasentrennung bzw. zur Sedimentation der dispergierten Stoffe.

Sprühbare Emulsionen erfordern niedrige Viskositäten (ca. 100 mPas), wie sie bei Feinemulsionen bereits bei Raumtemperatur realisierbar sind.

Sprühemulsionen haben in Bezug auf kosmetische und pharmazeutische Anwendungen gegenüber den klassischen als Lotionen, Cremes oder Salben konfektionierten Emulsionen entscheidende Vorteile. So geben Sprühemulsionen ein angenehmes Hautgefühl, können gut dosiert werden und sind vor Kontamination geschützt.

Feinemulsionen können gemäß dem Stand der Technik nach dem sogenannten "heiß/heiß Verfahren" hergestellt werden. Beim "heiß/heiß Verfahren" wird die Fettphase auf ca. 75°C erhitzt, vollständig geschmolzen und mit der ebenfalls ca. 75°C heißen Wasserphase unter höchstem mechanischen Energieeintrag vereinigt, um eine rasche Dispergierung sicherzustellen und eine hohe Feinheit des Systems zu erzielen. Dieses Verfahren erfordert einen hohen thermischen und mechanischen Energieaufwand.

Ein zweites Verfahren, das so genannte "Phasen-Inversions-Temperatur (PIT) Verfahren", ermöglicht den Verzicht auf intensive mechanische Dispergiervorgänge. Das "Phasen-Inversions-Temperatur Verfahren" beruht darauf, dass der O/W-Charakter eines hydrophilen, nichtionischen Tensides mit steigender Temperatur abnimmt und bei einer bestimmten Umschlagstemperatur zum W/O-Typ umschlägt. Beim Abkühlen erfolgt wiederum der Umschlag zum O/W-Typ. Die Umschlagstemperatur wird als "Phasen-Inversions-Temperatur (PIT)" bezeichnet.
Bei der Herstellung der Emulsion wird so verfahren, dass der eigentlich hydrophile Emulgator oberhalb seiner PIT in einer vorzugsweise polaren Ölphase gelöst und mit der Wasserphase emulgiert wird. Beim Abkühlen wird zunächst eine transparente Mikroemulsion durchlaufen, anschließend bilden sich ohne einen Homogenisierungsschritt hochdisperse, niedrigviskose Formulierungen.
Nachteilig am PIT-Verfahren ist, dass es auf ethoxylierte Tenside beschränkt ist, die eine ausreichend starke Temperaturabhängigkeit ihrer hydrophilen/lipophilen Eigenschaften zeigen.
Die Verwendung ethoxylierter Tenside für kosmetische und pharmazeutische Anwendungen ist jedoch problematisch, da diese unter dem Verdacht stehen, die Haut für schädliche Stoffe durchlässig zu machen und unter UV-Einwirkung nicht definierte, eventuell schädliche Stoffe zu bilden.

EP 0 699 125 offenbart ein PIT-Verfahren zur Herstellung von Dihydroxyaceton enthaltenden Emulsionen des Typs Öl-in-Wasser. Die durchschnittliche Teilchengröße der Ölphase dieser Emulsionen beträgt zwischen 100 und 1000 nm.

Aufgabe der Erfindung war es demnach, ein Verfahren zur Herstellung von Feinemulsionen zu entwickeln, wobei das Verfahren ohne den Einsatz ethoxylierter Produkte und ohne hohen thermischen und/oder mechanischen Energieaufwand auskommt.

Überraschenderweise wurde gefunden, dass Feinemulsionen, enthaltend mindestens einen W/O-Emulgator und mindestens eine hydrophile Komponente, ohne den Einsatz ethoxylierter Produkte und ohne hohen thermischen und/oder mechanischen Energieaufwand herstellbar sind.

Gegenstand der Erfindung ist ein Verfahren zur Herstellung von Feinemulsionen mit einer Teilchengröße im Bereich von 0,1 bis 10 Mikrometern, dadurch gekennzeichnet, dass eine W/O-Präemulsion, enthaltend mindestens einen W/O-Emulgator ausgewählt aus Sorbitolestern, Polyglycerolestern, Sorbitanestern, Fettsäureestern und/oder Dimethiconcopolyolen durch Zugabe mindestens einer hydrophilen Komponente ausgewählt aus Tensiden ohne Temperaturerhöhung in eine O/W-Feinemulsion überführt wird.

Die Menge an W/O-Emulgatoren, die die W/O-Präemulsion enthält, ist so bemessen, dass die fertige O/W-Feinemulsion bevorzugt 0,01 bis 10 Gew.-%, besonders bevorzugt 0,1 bis 8 Gew.-% und insbesondere bevorzugt 0,2 bis 4 Gew.-% an W/O-Emulgatoren enthält.

Die Menge an hydrophilen Komponenten, die der W/O-Präemulsion zugesetzt werden, ist so bemessen, dass die fertige O/W-Feinemulsion bevorzugt 0,01 bis 10 Gew.-%, besonders bevorzugt 0,1 bis 7 Gew.-% und insbesondere bevorzugt 0,3 bis 5 Gew.-% an hydrophilen Komponenten enthält.

Der nichtwässrige Anteil der Feinemulsionen, der sich weitgehend aus den W/O-Emulgatoren und dem Ölkörper zusammensetzt, beträgt bevorzugt 0,1 bis 95 % und besonders bevorzugt 0,5 bis 45 Gew.-%.

Als W/O-Emulgatoren bevorzugt sind Sorbitolester.

Bevorzugt werden solche Sorbitolester verwendet, die durch Umesterung von Sorbit, gegebenenfalls alkoxyliertem Sorbit, mit Fettsäuremethylestern oder Fettsäuretriglyceriden erhältlich sind, wobei die durch Umesterung erhaltenen Reaktionsprodukte gegebenenfalls anschließend alkoxyliert werden.
Im Falle von alkoxyliertem Sorbit als Umsetzungsprodukt handelt es sich vorzugsweise um ethoxylierten Sorbit. Der Gehalt an Ethoxylatgruppen beträgt bevorzugt 1 bis 90 -CH₂CH₂O-Gruppen pro Molekül Sorbit. Die Alkoxylierung des Sorbits kann nach den dem Fachmann bekannten Verfahren erfolgen.
Bei der Umesterung von Sorbit mit Fettsäuremethylestern arbeitet man jedoch vorzugsweise so, dass man zunächst die Umesterung mit Sorbit durchführt und anschließend nach den bekannten Verfahren das Reaktionsprodukt alkoxyliert.
Bei den Fettsäureresten der Fettsäuremethylester und Fettsäuretriglyceride handelt es sich bevorzugt um (C₈-C₂₂)-Reste, die geradkettig und/oder verzweigt und gesättigt und/oder ungesättigt sind. Beispiele hierfür sind Palmitinsäure, Stearinsäure, Laurinsäure, Linolsäure, Linolensäure, Isostearinsäure oder Ölsäure. Als Fettsäuretriglyceride eignen sich z.B. native tierische oder pflanzliche Öle, Fette und Wachse wie z.B. Rapsöl, Olivenöl, Palmkernöl, Sonnenblumenöl, Kokosöl, Leinöl, Ricinusöl, Sojabohnenöl, gegebenenfalls auch in raffinierter oder hydrierter Form. Da die natürlichen Fette, Öle und Wachse normalerweise Mischungen von Fettsäureresten mit unterschiedlicher Kettenlänge enthalten, gilt dies entsprechend auch für die daraus hergestellten Sorbitolester.

Insbesondere bevorzugt sind Sorbitolester auf Basis von Rapsöl. Diese sind für kosmetische und pharmazeutische Anwendungen besonders geeignet.

Die Herstellung der Sorbitolester aus Fettsäuremethylestern oder Fettsäuretriglyceriden kann entsprechend DE 197 27 950 und EP-A-1029586 erfolgen.

Die Umsetzung von Sorbit mit den Fettsäuretriglyceriden oder Methylestern erfolgt bevorzugt im Eintopfverfahren ohne Lösemittel bei Temperaturen von bevorzugt 120 - 140°C in Gegenwart eines alkalischen Katalysators. Das Molverhältnis von Sorbit zu Fettsäuremethylester beträgt bevorzugt 1:1 bis 1:2. Bei Verwendung von Fettsäuretriglyceriden beträgt das Molverhältnis von Sorbit zu Fettsäuretriglycerid 1:3,5 bis 1:4,5. Die Reaktionsdauer beträgt bevorzugt 12 bis 13 Stunden.
Bei der Verwendung von Fettsäuremethylestern wird das während der Reaktion entstehende Methanol abdestilliert. Da Sorbit üblicherweise als wässrige Lösung im Handel ist, wird das Wasser vor Einsatz vorteilhafterweise durch Destillation bei maximal 120°C unter vermindertem Druck entfernt.
Das Reaktionsprodukt dieser Umesterungsreaktion besteht neben Restmengen an nicht umgesetztem Sorbit im wesentlichen aus den Sorbitmonofettsäureestern und den Sorbitdifettsäureestern. Die entsprechenden Triester entstehen nur in untergeordneten Mengen. Bei der Verwendung von Fettsäuretriglyceriden als Ausgangsprodukt enthält das Reaktionsprodukt auch noch Mono- und Di-Fettsäureglycerid sowie nicht umgesetztes Triglycerid in Abhängigkeit von dem jeweils gewählten Molverhältnis der Ausgangsverbindungen.

Die hydrophob/hydrophilen Eigenschaften der Feinemulsionen lassen sich durch Auswahl der Fettsäurekomponente, gegebenenfalls durch den Ethoxylierungsgrad und/oder durch Zusatz von Neutralisationsmittel, insbesondere aber durch die Wahl der hydrophilen Komponente steuern.

Bei den als hydrophilen Komponenten verwendeten Tensiden kann es sich um anionische, kationische, zwitterionische, amphotere und/oder nichtionische Tenside handeln. Bevorzugt werden amphotere Tenside verwendet.

Als anionische Tenside eignen sich z.B. (C₁₀-C₂₀)-Alkyl- und Alkylencarboxylate, Alkylethercarboxylate, Fettalkoholsulfate, Fettalkoholethersulfate, Alkylamidsulfate und -sulfonate, Fettsäurealkylamidpolyglykolethersulfate, Alkansulfate, Alkansulfonate und Hydroxyalkansulfonate, Olefinsulfonate, Acylester von Isethionaten, α-Sulfofettsäureester, Alkylbenzolsulfonate, Alkylphenolglykolethersulfonate, Sulfosuccinate, Sulfobernsteinsäurehalbester und diester, Fettalkoholetherphosphate, Eiweiß-Fettsäure-Kondensationsprodukte, Alkylmonoglyceridsulfate und -sulfonate, Alkylglyceridethersulfonate, Fettsäuremethyltauride, Fettsäuresarkosinate, Sulforicinoleate, Amphoacetate- oder -glycinate und/oder Acylglutamate. Die genannten Verbindungen bzw. deren Mischungen werden bevorzugt in Form ihrer wasserlöslichen oder in Wasser dispergierbaren Salze, beispielsweise der Natrium-, Kalium-, Magnesium-, Ammonium-, Mono-, Di- und Triethanolammonium- sowie analogen Alkylammoniumsalze, verwendet.

Als kationische Tenside eignen sich z.B. quartäre Ammoniumsalze, wie z.B. Di-((C₁₀-C₂₄)-Alkyl)-dimethyl-ammonium-chlorid und -bromid, vorzugsweise Di-((C₁₂-C₁₈)-Alkyl)-dimethyl-ammonium-chlorid und -bromid, ((C₁₀-C₂₄)-Alkyl)-dimethylethylammonium-chlorid und -bromid; ((C₁₀-C₂₄)-Alkyl)-trimethyl-ammonium-chlorid und -bromid, vorzugsweise Cetyl-trimethyl-ammonium-chlorid oder -bromid und ((C₂₀-C₂₂)-Alkyl)-trimethyl-ammonium-chlorid und -bromid; ((C₁₀-C₂₄)-Alkyl)-dimethylbenzyl-ammonium-chlorid und -bromid, vorzugsweise ((C₁₂-C₁₈)-Alkyl)-dimethylbenzyl-ammonium-chlorid; N-((C₁₀-C₁₈)-Alkyl)-pyridinium-chlorid und -bromid, vorzugsweise N-((C₁₂-C₁₆)-Alkyl)-pyridinium-chlorid und -bromid; N-((C₁₀-C₁₈)-Alkyl)-isochinolinium-chlorid, -bromid und -monoalkylsulfat; N-((C₁₂-C₁₈)-Alkyl)-polyoylaminoformylmethyl-pyridinium-chlorid; N-((C₁₂-C₁₈)-Alkyl)-N-methyl-morpholinium-chlorid, -bromid und -monoalkylsulfat; N-((C₁₂-C₁₈-Alkyl)-N-ethyl-morpholinium-chlorid, -bromid und -monoalkylsulfat; ((C₁₆-C₁₈)-Alkyl)-pentaoxethyl-ammonium-chlorid; Diisobutylphenoxyethoxyethyldimethylbenzylammonium-chlorid; Salze des N,N-Diethylaminoethylstearylamids und -oleylamids mit Salzsäure, Essigsäure, Milchsäure, Zitronensäure, Phosphorsäure; N-Acyl-aminoethyl-N,N-diethyl-N-methyl-ammoniumchlorid, -bromid und -monoalkylsulfat; und/oder N-Acylaminoethyl-N,N-diethyl-N-benzyl-ammonium-chlorid, -bromid oder -monoalkylsulfat, wobei Acyl vorzugsweise für Stearyl oder Oleyl steht.

Als amphotere Tenside eignen sich bevorzugt N-((C₁₂-C₁₈)-Alkyl)-β-aminopropionate und N-((C₁₂-C₁₈)-Alkyl)-β-iminodipropionate als Alkali- und Mono-, Di- und Trialkylammoniumsalze; N-Acylaminoalkyl-N,N-dimethyl-acetobetain, vorzugsweise N-(C₈-C₁₈-Acyl)aminopropyl-N,N-dimethylacetobetain; ((C₁₂-C₁₈)-Alkyl)-dimethyl-sulfopropyl-betain; Amphotenside auf Basis Imidazolin (Miranol^{®}, Steinapon^{®}), vorzugsweise das Natrium-Salz des 1-(β-Carboxy-methyloxyethyl)-1-(carboxymethyl)-2-lauryl-imidazoliniums; und/oder Acylglutamate. Besonders bevorzugt eignen sich die Acylglutamate.

Als nichtionische Tenside eignen sich beispielsweise Fettalkoholethoxylate (Alkylpolyethylenglykole); Alkylphenolpolyethylenglykole; Alkylmercaptanpolyethylenglykole; Fettaminethoxylate (Alkylaminopolyethylenglykole); Fettsäureethoxylate (Acylpolyethylenglykole); Polypropylenglykolethoxylate (Poloxamere); Fettsäureamidpolyethylenglykole; N-Alkyl- und N-Alkoxypolyhydroxyfettsäureamide, insbesondere Fettsäure-N-methylglucamide; Saccharoseester; Polyglykolether; Alkylpolyglycoside; Phosphorsäureester (Mono-, Di- und Triphosphorsäureester ethoxyliert und nicht-ethoxyliert); und/oder Aminoxide, wie z.B. ((C₁₂-C₁₈)-Alkyl)-dimethylaminoxid und Fettsäureamidoalkyldimethylaminoxid.

Die durch das erfindungsgemäße Verfahren hergestellten Feinemulsionen können auch Co-Emulgatoren, Soil Release Polymere und/oder Säure/Lauge Komponenten enthalten.

Als nichtionogene Co-Emulgatoren eignen sich Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe; (C₁₂-C₁₈)-Fettsäuremono- und -diester von Anlagerungsprodukten von 1 bis 30 Mol Ethylenoxid an Glycerin; Glycerinmono- und -diester und Sorbitanmono- und -diester von gesättigten und ungesättigten Fettsäuren mit 6 bis 22 Kohlenstoffatomen und deren Ethylenoxidanlagerungsprodukten; Anlagerungsprodukte von 15 bis 60 Mol Ethylenoxid an Rizinusöl und/oder gehärtetes Rizinusöl; und/oder Polyol- und insbesondere Polyglycerinester, wie z.B. Polyglycerinpolyricinoleat und Polyglycerinpoly-12-hydroxystearat.
Bei den Anlagerungsprodukten von Ethylenoxid und/oder Propylenoxid an Fettalkohole; Fettsäuren; Alkylphenole; Glycerin-mono- und -diester; Sorbitanmono- und -diester von Fettsäuren; und Rizinusöl handelt es sich um bekannte im Handel erhältliche Produkte. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht.

Bevorzugt eignen sich als Soil Release Polymere Oligoester, erhalten durch Polykondensation von 40 bis 52 mol %, vorzugsweise 45 bis 50 mol %, einer oder mehrerer Dicarbonsäuren und/oder deren Ester;10 bis 40 mol %, vorzugsweise 20 bis 35 mol %, Ethylenglykol und/oder Propylenglykol; 3 bis 20 mol %, vorzugsweise 10 bis 15 mol %, Polyethylenglykol; 0 bis 10 mol % eines wasserlöslichen Anlagerungsproduktes von 5 bis 80 mol eines Alkylenoxids an 1 mol (C₁-C₂₄)-Alkohole, (C₆-C₁₈)-Alkylphenole oder (C₈-C₂₄)-Alkylamine; und 0 bis 10 mol % eines oder mehrerer Polyole mit 3 bis 6 Hydroxylgruppen.

Im Falle der Säure/Lauge Komponente sind bevorzugte Säuren und Laugen Ethercarbonsäuren und deren Salze oder Phosphorsäure-mono- und/oder -di-ester und deren neutralisierte Salze.

Als Ölkörper für die Feinemulsionen eignen sich bevorzugt Guerbetalkohole mit 6 bis 18, vorzugsweise 8 bis 10, Kohlenstoffatomen; Ester von linearen (C₆-C₁₃)-Fettsäuren mit linearen (C₆-C₂₀)-Fettalkoholen; Ester von verzweigten (C₆-C₁₃)-Carbonsäuren mit linearen (C₆-C₂₀)-Fettalkoholen, Ester von linearen (C₆-C₁₈)-Fettsäuren mit verzweigten Alkoholen, insbesondere 2-Ethylhexanol; Ester von linearen und/oder verzweigten Fettsäuren mit mehrwertigen Alkoholen (wie z.B. Dimerdiol oder Trimerdiol) und/oder Guerbetalkoholen; Triglyceride auf Basis (C₆-C₁₀)-Fettsäuren; pflanzliche Öle; verzweigte primäre Alkohole; substituierte Cyclohexane; Guerbetcarbonate; Dialkylether; und/oder aliphatische bzw. aromatische Kohlenwasserstoffe.

Als Hilfs- und Zusatzstoffe können die Feinemulsionen z.B. Überfettungsmittel; Fette; Wachse; Stabilisatoren; biogene Wirkstoffe; Lichtschutzstoffe (UV-Lichtschutzfilter, Pigmente, Mikropigmente), Antioxidantien; Hydrotrope; Solubilisatoren; Konsistenzgeber; Kationpolymere; Glycerin; Konservierungsmittel; Dispergiermittel; Eiweißderivate, wie z.B. Gelatine, Collagenhydrolysate, Polypeptide auf natürlicher und synthetischer Basis, Eigelb; Lecithin; Lanolin und Lanolinderivate; Fettalkohole; Silicone; deodorierende Mittel; Stoffe mit keratolytischer und keratoplastischer Wirkung; Enzyme; Trägersubstanzen; feuchtigkeitsspendende Stoffe; antimikrobiell wirkende Agentien; und/oder Farb- und Duftstoffe enthalten.

Als Überfettungsmittel eignen sich z.B. polyethoxylierte Lanolinderivate, Lecithinderivate, Polyolfettsäureester, Monoglyceride und Fettsäurealkanolamide, wobei die letzteren gleichzeitig als Schaumstabilisatoren dienen. Typische Beispiele für Fette sind Glyceride. Als Wachse kommen u.a. Bienenwachs, Paraffinwachs oder Mikrowachse, gegebenenfalls in Kombination mit hydrophilen Wachsen, wie z.B. Cetylstearylalkohol, in Frage.

Als UV-Filter eignen sich z.B. 4-Aminobenzoesäure; 3-(4'-Trimethylammonium)benzyliden-bornan-2-on-methylsulfat; 3,3,5-Trimethyl-cyclohexylsalicylat; 2-Hydroxy-4-methoxybenzophenon; 2-Phenylbenzimidazol-5-sulfonsäure und ihre Kalium-, Natrium- und Triethanolaminsalze; 3,3'-(1,4-Phenylendimethin)-bis-(7,7-dimethyl-2-oxobicyclo[2.2.1]-heptan-1-methansulfonsäure und ihre Salze; 1-(4-tert.-Butylphenyl)-3-(4-methoxyphenyl)propan-1,3-dion, 3-(4'-Sulfo)-benzyliden-bornan-2-on und seine Salze; 2-Cyan-3,3-diphenyl-acrylsäure-(2-ethylhexylester); Polymer von N-[2(und 4)-(2-oxoborn-3-ylidenmethyl)benzyl]-acrylamid; 4-Methoxy-zimtsäure-2-ethyl-hexylester; ethoxyliertes Ethyl-4-amino-benzoat; 4-Methoxy-zimtsäureisoamylester; 2,4,6-Tris-[p-(2-ethylhexyloxycarbonyl)anilino]-1,3,5-triazin; 2-(2H-benzotriazol-2-yl)-4-methyl-6-(2-methyl-3-(1,3,3,3-tetramethyl-1-(trimethylsilyloxy)-disiloxanyl)propyl)phenol; 4,4'-[(6-[4-((1,1-dimethylethyl)-amino-carbonyl)phenylamino]-1,3,5-triazin-2,4-yl)diimino]bis-(benzoesäure-2-ethylhexylester); 3-(4'-Methylbenzyliden)-D,L-Campher; 3-Benzyliden-Campher; Salicylsäure-2-ethylhexylester; 4-Dimethylaminobenzoesäure-2-ethylhexylester; Hydroxy-4-methoxy-benzophenon-5-sulfonsäure (Sulisobenzonum) und das Natriumsalz; und/oder 4-lsopropylbenzylsalicylat.

Als Pigmente/Mikropigmente können z.B. mikrofeines Titandioxid und Zinkoxid eingesetzt werden.

Als Antioxidantien eignen sich beispielsweise Superoxid-Dismutase, Tocopherol (Vitamin E) und Ascorbinsäure (Vitamin C).

Als Stabilisatoren können Metallsalze von Fettsäuren, wie z.B. Magnesium-, Aluminium- und/oder Zinkstearat eingesetzt werden.

Unter biogenen Wirkstoffen sind beispielsweise Pflanzenextrakte und Vitaminkomplexe zu verstehen.

Als Konservierungsmittel eignen sich beispielsweise Phenoxyethanol, Formaldehydlösung, Parabene, Pentandiol oder Sorbinsäure.

Als Farbstoffe können die für kosmetische Zwecke geeigneten und zugelassenen Substanzen verwendet werden.

Als Verdickungs- und Dispergiermittel besonders geeignet sind Ethylenglykolester von Fettsäuren mit 14 bis 22, besonders bevorzugt 16 bis 22, Kohlenstoffatomen, insbesondere Mono- und Di-ethylenglykolstearat. Ebenfalls bevorzugt geeignet sind Stearinmonoethanolamid, Stearindiethanolamid, Stearinisopropanolamid, Stearinmonoethanolamidstearat, Stearylstearat, Cetylpalmitat, Glycerylstearat, Stearamiddiethanolamiddistearat, Stearamidmonoethanolamidstearat, N,N-Dihydrocarbyl-(C₁₂-C₂₂)-amidobenzoesäure und deren lösliche Salze, N,N-Dihydrocarbyl-(C₁₆-C₁₈)-amidobenzoesäure und deren lösliche Salze und N,N-di(C₁₆-C₁₈)-amidobenzoesäure und deren Derivate. Weiterhin besonders geeignet sind Polyacrylate und Carbomere, insbesondere solche wasserlöslicher oder wasserquellbarer Copolymerisate auf Basis von Acrylamidoalkylsulfonsäuren und N-Vinylcarbonsäureamiden.

Als Solubilisatoren eignen sich prinzipiell alle ein- oder mehrwertigen Alkohole und ethoxylierten Alkohole. Bevorzugt werden Alkohole mit 1 bis 4 Kohlenstoffatomen, wie z.B. Ethanol, Propanol, Isopropanol, n-Butanol und Iso-Butanol, Glycerin und deren Mischungen eingesetzt. Weiterhin bevorzugt sind Polyethylenglykole mit einer relativen Molekülmasse unter 2000. Insbesondere bevorzugt sind Polyethylenglykole mit einer relativen Molekülmasse zwischen 200 und 600 in Mengen bis zu 45 Gew.-% und Polyethylenglykole mit einer relativen Molekülmasse zwischen 400 und 600 in Mengen von 0,5 bis 15 Gew.-%. Weitere geeignete Lösungsmittel sind beispielsweise Triacetin (Glycerintriacetat) und 1-Methoxy-2-propanol.

Als Trägermaterialien eignen sich z.B. pflanzliche Öle, natürliche und gehärtete Öle, Wachse, Fette, Wasser, Alkohole, Polyole, Glycerol, Glyceride, flüssige Paraffine, flüssige Fettalkohole, Sterole, Polyethylenglykole, Cellulose und Cellulose-Derivate.

Als fungizide Wirkstoffe eignen sich bevorzugt Ketoconazol, Oxiconazol, Bifonazole, Butoconazole, Cloconazole, Clotrimazole, Econazole, Enilconazole, Fenticonazole, Isoconazole, Miconazole, Sulconazole, Tioconazole, Fluconazole, Itraconazole, Terconazole, Naftifine und Terbinafine, Zn-Pyrethion und Octopyrox.

Als deodorierende Stoffe können z.B. Allantoin und Bisabolol in Gewichtsmengen von 0,0001 % bis 10 % eingesetzt werden.

Geeignete kationische Polymere sind z.B. kationische Cellulosederivate; kationische Stärke; Copolymere von Diallylammoniumsalzen und Acrylamiden; quaternierte Vinylpyrrolidon/ Vinylimidazol-Polymere; Kondensationsprodukte von Polyglykolen und Aminen; quaternierte Kollagenpolypeptide; quaternierte Weizenpolypeptide; Polyethylenimine; kationische Siliconpolymere, wie z.B. Amidomethicone; Copolymere der Adipinsäure und Dimethylaminohydroxy-propyldiethylentriamin; Polyaminopolyamid und kationische Chitinderivate, wie beispielsweise Chitosan.

Geeignete Siliconverbindungen sind beispielsweise Dimethylpolysiloxan, Methylphenylpolysiloxan, cyclische Silicone und amino-, fettsäure-, alkohol-, polyether-, epoxy-, fluor- und/oder alkylmodifizierte Siliconverbindungen, sowie Polyalkylsiloxane, Polyalkylarylsiloxane, Polyethersiloxan-Copolymere, wie in US 5104 645 und den darin zitierten Schriften beschrieben, die bei Raumtemperatur sowohl flüssig als auch harzförmig vorliegen können.

Die Mittel können mit konventionellen Ceramiden, Pseudoceramiden, Fettsäure-N-alkylpolyhydroxyalkylamiden, Cholesterin, Cholesterinfettsäureestern, Fettsäuren, Triglyceriden, Cerebrosiden, Phospholipiden und ähnlichen Stoffen abgemischt werden.

Als feuchtigkeitsspendende Substanz stehen beispielsweise Isopropylpalmitat, Glycerin und/ oder Sorbitol zu Verfügung, die in den Gewichtsmengen 0,1 bis 50 % eingesetzt werden können.

Der Gesamtanteil der Hilfs- und Zusatzstoffe beträgt, bezogen auf die fertigen Feinemulsionen, bevorzugt 1 bis 10 Gew.-%, besonders bevorzugt 2 bis 5 Gew.-%.

Im Hinblick auf die eingangs schon erläuterte Problematik ethyloxierter Produkte sind die Feinemulsionen vorteilhafterweise frei von ethoxylierten Komponenten (W/O-Emulgatoren, hydrophilen Komponenten etc).

Bei den Feinemulsionen handelt es sich bevorzugt um kosmetische und pharmazeutische Sprühemulsionen, z.B. solche zur Pflege der Haut. Beispielhaft seien Bodylotions, After-Sun Lotions, Sonnenschutzmittel, pharmazeutisch wirksame Wirkstoffe enthaltende Lotionen, Deo-Sprays und Lotionen für die Herstellung von feuchtigkeitsgetränkten Reinigungs- und Pflegetüchern genannt.

Gegebenenfalls werden der W/O-Präemulsion und/oder der O/W-Feinemulsion die schon oben beschriebenen Zusatz- und Hilfsstoffen zugesetzt. Der Gewichtsanteil an Zusatz- und Hilfsstoffen beträgt bevorzugt, bezogen auf die fertige Feinemulsion, 0,1 bis 10 Gew.-%.

Vorteilhafterweise werden den Feinemulsionen bei der Herstellung keine ethoxylierten Komponenten (W/O-Emulgatoren, hydrophile Komponenten etc.) zugesetzt.

Die nachfolgenden Beispiele dienen der Erläuterung der Erfindung, ohne sie jedoch darauf einzuschränken. Bei den Prozentangaben handelt es sich um Gewichtsprozente.

### Beispiel 1: Sprühbare Pflege-O/W-Mikroemulsion (keine ethoxylierten Komponenten)

| | |
|---|---|
| Emulsogen^{®} SRO (Clariant GmbH) | 4,0 % |
| (Rapsöl Sorbitolester) | |
| Sojaöl | 2,0 % |
| Mandelöl | 0,8 % |
| Avocadoöl | 0,8 % |
| Jojobaöl | 0,4 % |
| Cutina^{®} GMS (Cognis GmbH) | 1,0 % |
| Glycerylstearat | |
| Carbopol^{®} 980 (BF Goodrich) | 0,2 % |
| (Carbomer) | |
| Wasser | 44,7% |
| Natriumhydroxid | 0,8 % |
| (10 % wässrige Lösung) | |
| Wasser | 44,9 % |
| Hostapon^{®} CLG (Clariant GmbH) | 0,6 % |
| (Natriumlauroylglutamat) | |

Herstellung:
1) Emulgator und Öle auf ca. 80°C erwärmen
2) Carbomer hinzufügen
3) 1. Teil Wasser mit Natronlauge auf ca. 80°C erwärmen
4) 1. Teil Wasser zur Ölphase unter Rühren mit Dispersionswerkzeug hinzugeben und ca. 2 Minuten zu einer W/O Emulsion dispergieren
5) 2. Teil Wasser mit dem Acylglutamat mischen und kalt unter Rühren hinzugeben, ca. 2 Stunden nachrühren

### Beispiel 2: Sprühbare Pflege-Emulsion (keine ethoxylierten Komponenten, kalt herstellbar)

| | |
|---|---|
| Emulsogen^{®} SRO (Clariant GmbH) | 4,0 % |
| (Rapsöl Sorbitolester) | |
| Sojaöl | 2,0 % |
| Mandelöl | 0,8 % |
| Avocadoöl | 0,8 % |
| Jojobaöl | 0,4 % |
| Isopropylpalmitat | 1,0 % |
| Carbopol^{®} 980 (BF Goodrich) | 0,2 % |
| (Carbomer) | |
| Wasser | 44,7 % |
| Natriumhydroxid | 0,8 % |
| (10 % wässrige Lösung) | |
| Wasser | 44,9 % |
| Hostapon^{®} CLG (Clariant GmbH) | 0,6 % |
| (Natriumlauroylglutamat) | |

Herstellung:
1) Emulgator, Öle und Carbomer mischen
2) 1. Teil Wasser mit Natronlauge bei Raumtemperatur zu der Ölphase unter Rühren mit Dispersionswerkzeug hinzugeben und ca. 2 Minuten zu einer W/O Emulsion dispergieren
3) 2. Teil Wasser mit dem Acylglutamat mischen und kalt unter Rühren hinzugeben, ca. 2 Stunden nachrühren

### Beispiel 3: Sprühbare After-Sun-Emulsion

| | |
|---|---|
| Emulsogen^{®} SRO (Clariant GmbH) | 4,0 % |
| (Rapsöl Sorbitolester) | |
| Sojaöl | 0,5 % |
| Mandelöl | 0,2 % |
| Avocadoöl | 0,2 % |
| Jojobaöl | 0,1 % |
| Emulsogen^{®} HCO 040 (Clariant GmbH) | 2,0 % |
| (PEG-40 Hydrogenated Castor Oil) | |
| Myritol^{®} 318 | 1,0 % |
| (Caprylic/Carpic Triglyceride) | |
| Cetiol^{®} SN (Cognis GmbH) | 1,0 % |
| (Cetearyl Isononanoat) | |
| Mineralöl niedrigviskos | 1,0 % |
| Cutina^{®} GMS (Cognis GmbH) | 0,2 % |
| (Glyceryl Stearate) | |
| Isopropyl Palmitat | 1,0 % |
| Lanette^{®} O (Cognis GmbH) | 0,2 % |
| (Cetearylalkohol) | |
| Carbopol^{®} 980 (BF Goodrich) | 0,2 % |
| (Carbomer) | |
| Wasser | 38,0 % |
| Natriumhydroxid | 0,7 % |
| (10 % wässrige Lösung) | |
| Wasser | 39,4 % |
| Hostapon^{®} CLG (Clariant GmbH) | 0,6 % |
| (Natriumlauroylglutamat) | |
| Glycerin | 5,0 % |
| Panthenol | 0,5 % |
| Tocopherylacetat | 0,2 % |
| Ethanol | 4,0 % |

Herstellung:
1) Emulgator und Öle auf ca. 80°C erwärmen
2) Carbomer hinzufügen
3) 1. Teil Wasser mit Natronlauge auf ca. 80°C erwärmen
4) 1. Teil Wasser zu der Ölphase unter Rühren mit Dispersionswerkzeug hinzugeben und ca. 2 Minuten zu einer W/O Emulsion dispergieren
5) 2. Teil Wasser mit dem Acylglutamat mischen und mit Glycerin und Panthenol kalt unter Rühren hinzugeben, ca. 2 Stunden nachrühren.
6) Tocopherylacetat und Ethanol zugeben, ca. 0,5 Stunden nachrühren.

## Patentansprüche

1. Verfahren zur Herstellung von Feinemulsionen mit einer Teilchengröße im Bereich von 0,1 bis 10 Mikrometern, **dadurch gekennzeichnet, dass** eine W/O-Präemulsion, enthaltend mindestens einen W/O-Emulgator ausgewählt aus Sorbitolestern, Polyglycerolestern, Sorbitanestern, Fettsäureestern und/oder Dimethiconcopolyolen durch Zugabe mindestens einer hydrophilen Komponente ausgewählt aus Tensiden ohne Temperaturerhöhung in eine O/W-Feinemulsion überführt wird.

2. Verfahren nach Anspruch 1, **dadurch gekennzeichnet, dass** die Menge an W/O-Emulgatoren, die die W/O-Präemulsion enthält, so bemessen ist, dass die O/W-Feinemulsion 0,01 bis 10 Gew.-% an W/O-Emulgatoren enthält.

3. Verfahren nach Anspruch 1 oder 2, **dadurch gekennzeichnet, dass** die Menge an hydrophilen Komponenten, die der W/O-Präemulsion zugesetzt werden, so bemessen ist, dass die O/W-Feinemulsion 0,01 bis 10 Gew.-% an hydrophilen Komponenten enthält.

4. Verfahren nach mindestens einem der Ansprüche 1 bis 3, **dadurch gekennzeichnet, dass** es sich bei den W/O-Emulgatoren um Sorbitolester handelt.

5. Verfahren nach mindestens einem der Ansprüche 1 bis 4, **dadurch gekennzeichnet, dass** es sich bei den Tensiden um amphotere Tenside handelt.

6. Verfahren nach mindestens einem der Ansprüche 1 bis 5, **dadurch gekennzeichnet, dass** der W/O-Präemulsion und/oder der O/W-Feinemulsion gegebenenfalls noch Zusatz- und Hilfsstoffe zugesetzt werden.

7. Verfahren nach mindestens einem der Ansprüche 1 bis 6, **dadurch gekennzeichnet, dass** bei der Herstellung der Feinemulsion keine ethoxylierten Komponenten verwendet werden.

## Claims

1. A process for the preparation of fine emulsions having a particle size in the range from 0.1 to 10 micrometers, which comprises converting a W/O preemulsion comprising at least one W/O emulsifier selected from sorbitol esters, polyglycerol esters, sorbitan esters, fatty acid esters and/or dimethicone copolyols into an O/W fine emulsion by adding at least one hydrophilic component selected from surfactants without increasing the temperature.

2. The process as claimed in claim 1, wherein the amount of W/O emulsifiers which the W/O preemulsion comprises is measured such that the O/W fine emulsion comprises 0.01 to 10% by weight of W/O emulsifiers.

3. The process as claimed in claim 1 or 2, wherein the amount of hydrophilic components which are added to the W/O preemulsion is measured such that the O/W fine emulsion comprises 0.01 to 10% by weight of hydrophilic components.

4. The process as claimed in at least one of claims 1 to 3, wherein the W/O emulsifiers are sorbitol esters.

5. The process as claimed in at least one of claims 1 to 4, wherein the surfactants are amphoteric surfactants.

6. The process as claimed in at least one of claims 1 to 5, wherein additives and auxiliaries are also, where appropriate, added to the W/O preemulsion and/or the O/W fine emulsion.

7. The process as claimed in at least one of claims 1 to 6, wherein no ethoxylated components are used during the preparation of the fine emulsion.

## Revendications

1. Procédé de préparation d'émulsions fines ayant une taille de particule de l'ordre de 0,1 à 10 microns,
**caractérisé en ce qu'**
on transforme une pré-émulsion E/H, contenant au moins un émulsifiant E/H, choisi parmi les esters de sorbitol, les esters de polyglycérol, les esters de sorbitane, les esters d'acides gras et/ou les copolyols de diméthicone, en une émulsion fine H/E en ajoutant au moins un composant hydrophile choisi parmi les tensioactifs sans augmenter la température.

2. Procédé selon la revendication 1,
**caractérisé en ce que**
la quantité d'émulsifiants E/H, qui contient une pré-émulsion E/H, est ajustée pour que l'émulsion fine H/E contienne de 0,01 à 10 % en poids d'émulsifiants H/E.

3. Procédé selon la revendication 1 ou 2,
**caractérisé en ce que**
la quantité de composants hydrophiles ajoutés à la pré-émulsion E/H, est ajustée pour que l'émulsion fine H/E contienne de 0,01 à 10 % en poids de composants hydrophiles.

4. Procédé selon au moins l'une quelconque des revendications 1 à 3,
**caractérisé en ce que**
les émulsifiants E/H sont des esters de sorbitol.

5. Procédé selon au moins l'une quelconque des revendications 1 à 4,
**caractérisé en ce que**
les tensioactifs sont des tensioactifs amphotères.

6. Procédé selon au moins l'une quelconque des revendications 1 à 5,
**caractérisé en ce qu'**
on ajoute encore, le cas échéant, des additifs et des excipients à la pré-émulsion E/H et/ou à l'émulsion fine H/E.

7. Procédé selon au moins l'une quelconque des revendications 1 à 6,
**caractérisé en ce qu'**
on n'utilise pas de composants éthoxylés lors de la préparation de l'émulsion fine.
